# FASCICULE DE BREVET EUROPEEN

(11) **EP 1 386 916 B1**
(45) Date de publication et mention de la délivrance du brevet: **18.01.2006**
(21) Numéro de dépôt: 03291763.5
(22) Date de dépôt: 16.07.2003
(51) Int. Cl.: C07D 233/58, C07D 231/12, C07D 295/02

(54) **Procédé de monométhylation d'hétérocycles azotés.**
Verfahren zur Monomethylierung von stickstoffhaltigen Heterocyclen
Process for the monomethylation of nitrogen containing heterocycles

(30) Priorité: 01.08.2002 FR 0209820
(43) Date de publication de la demande: 04.02.2004
(73) Titulaire: ISOCHEM, 75004 Paris (FR)
(72) Inventeur: Borredon, Elisabeth, 31170 Tournefeuille (FR); Chabaud, Bernard, 84200 Carpentras (FR); Gaset, Antoine, 31000 Toulouse (FR); Ouk, Samedy, Los Angeles, CA 90020 (US); Thiebaud-Roux, Sophie, 31240 L'Union (FR)
(74) Mandataire: Ahner, Francis

(56) Documents cités:
- WO-A-96/08537
- DATABASE CA [en ligne] CHEMICAL ABSTRACTS SERVICE, COLUMBUS, OHIO, US; KISO, HIROYUKI ET AL: "Preparation of N-methylimidazole derivatives" retrieved from STN Database accession no. 127:50642 XP002237275 -& JP 09 169737 A (TOSOH CORP., JAPAN) 30 juin 1997 (1997-06-30)
- LISSEL M: "LIEBIGS ANNALEN DER CHEMIE, VERLAG CHEMIE GMBH. WEINHEIM, DE" LIEBIGS ANNALEN DER CHEMIE, VERLAG CHEMIE GMBH. WEINHEIM, DE, 1987, pages 77-79, XP002125053 ISSN: 0170-2041

## Description

La présente invention concerne un procédé de mono méthylation d'hétérocycles azotés. De tels hétérocycles, comme la famille des azoles, ont des applications dans divers domaines tels que l'agrochimie, la pharmacie, les biotechnologies, les peintures et les colorants.

L'homme du métier connaît déjà des procédés de N-méthylation d'hétérocycles azotés. Certains procédés consistent à alkyler les hétérocycles azotés avec des halogènures d'alkyle ou des sulfates d'alkyle, ces procédés présentent de nombreux inconvénients. Certains réactifs comme le sulfate de diméthyle sont très toxiques. De plus, le produit obtenu nécessite un procédé complexe de purification. Des procédés utilisant des réactifs moins toxiques pour l'environnement ont alors été proposés. Ainsi, plusieurs procédés de méthylation par le carbonate de diméthyle ont été envisagés.

Les auteurs d'un article paru dans Liebigs Ann. Chem. (Liebigs Ann. Chem. 1987, 1, 77) décrivent un procédé de N-méthylation de l'imidazole, du benzimidazole et de leurs dérivés par réaction de l'hétérocycle azoté avec du carbonate de diméthyle en présente d'une base (du carbonate de potassium) et d'un catalyseur de transfert de phase (l'éther couronne [18-6]). Les inconvénients d'une telle synthèse sont le coût élevé et la toxicité de l'éther couronne. De plus, une opération de séparation du catalyseur du milieu réactionnel est nécessaire en fin de réaction.

Un procédé décrit dans le brevet WO 96/08537, ne faisant pas intervenir d'éther couronne a alors été proposé. Ce procédé consiste à produire des pigments organiques en méthylant des hétérocycles par du carbonate de diméthyle, en présence de carbonates ou d'hydroxydes alcalins. La réaction est effectuée à une température comprise entre 80°C et 150°C, sous pression atmosphérique. Or, même en présence d'un catalyseur, le rendement reste faible.

Un autre procédé, décrit dans le brevet JP 9169737, propose un procédé de synthèse d'imidazoles N-méthylés par réaction, à une température comprise entre 120°C et 200°C, de préférence à 160°C, de l'imidazole avec du carbonate de diméthyle. Le procédé décrit ne se limite qu'à la synthèse de dérivés de méthyl imidazoles à partir de composés qui ont des points d'ébullition aux environs de 250°C. Des essais effectués avec des hétérocycles ayant des points d'ébullition plus bas montrent que ce procédé n'est pas généralisable à tous les hétérocycles azotés, et notamment aux hétérocycles azotés ayant un point d'ébullition inférieur à 190°C.

L'homme du métier est donc toujours à la recherche d'un procédé de N-méthylation d'hétérocycles azotés, ce procédé devant être simple de mise en oeuvre et devant être généralisable à plusieurs familles d'hétérocycles azotés. Dans le cas où l'hétérocycle azoté possède plusieurs atomes d'azotes, liés chacun à un atome d'hydrogène, l'homme du métier est à la recherche d'un procédé sélectif, c'est-à-dire un procédé ne permettant de synthétiser que le produit mono-méthylé.

La présente invention concerne un tel procédé.

Elle concerne un procédé de mono-méthylation d'hétérocycles azotés contenant au moins un atome d'azote lié à un atome d'hydrogène par réaction dudit hétérocycle avec du carbonate de diméthyle, caractérisé en ce que la réaction est effectuée à une température comprise entre 100°C et 200°C, à une pression comprise entre 0,93.10⁵Pa et 1,07.10⁵Pa et en ce que le méthanol, produit au cours de la réaction, est soutiré en continu.

Ce procédé, effectué à une température comprise entre 100°C et 200°C et aux environs de la pression atmosphérique, présente l'avantage d'être simple de mise en oeuvre. De plus, le fait de soutirer le méthanol en continu, c'est-à-dire de le distiller au fur et à mesure de sa formation, permet de contrôler la température du milieu réactionnel. Cela présente plusieurs avantages. Le procédé est ainsi applicable à plusieurs familles d'hétérocycles azotés et pas seulement aux hétérocycles ayant un point d'ébullition élevé, de l'ordre de 250°C. Cela permet également d'ajouter du carbonate de diméthyle en grande quantité dans le milieu réactionnel sans en faire baisser la température. La cinétique de la réaction étant fonction de la quantité de carbonate de diméthyle dans le milieu réactionnel, la réaction est effectuée avec une très bonne cinétique.

Ce procédé s'applique non seulement à des hétérocycles ayant une température d'ébullition de l'ordre de 190°C ou supérieure à 190°C, de l'ordre de 250°C, mais aussi à des hétérocycles ayant des températures d'ébullition plus basses, de l'ordre de 120°C.

De façon générale, le procédé s'applique à des hétérocycles ayant une température d'ébullition supérieure ou égale à 120°C.

De préférence, les hétérocycles azotés sont choisis parmi les azoles et leurs dérivés benzéniques, l'indoline, la pyrazolidine, la morpholine, la pipérazine et l'azépine.

On appelle azoles des composés hétérocycliques à cinq chaînons dont au moins un atome d'azote est lié à un atome d'hydrogène. On pourra citer comme azoles contenant un atome d'azote, l'indole et le carbazole et comme azoles contenant deux atomes d'azote l'imidazole, le benzimidazole, le pyrazole et l'indazole. Les azoles contenant trois atomes d'azote sont notamment les triazoles et benzotriazoles et l'azole contenant cinq atomes d'azote est le pentazole.

La quantité de carbonate de diméthyle utilisée est comprise entre 1 et 5 moles par mole d'hétérocycle azoté, et de préférence entre 1,2 et 3 moles par mole d'hétérocycle azoté.

Le carbonate de diméthyle est généralement ajouté progressivement dans le milieu réactionnel, avec un débit compris entre 0,001 mol/mol de substrat.h et 1 mol/mol de substrat.h, le substrat étant l'hétérocycle azoté.

La réaction est effectuée à une température comprise entre 100°C et 200°C et de préférence entre 120°C et 180°C.

La réaction est effectuée à une pression comprise entre 0,93.10⁵Pa et 1,07.10⁵Pa, soit à une pression comprise entre 700mm Hg et 800mm Hg. Généralement, la pression atmosphérique locale est comprise dans cette gamme et la réaction est effectuée à cette pression.

L'hétérocycle azoté est mono-méthylé sur l'atome d'azote lié à un atome d'hydrogène. Lorsque l'hétérocycle azoté contient plus d'un atome d'azote, c'est-à-dire au moins deux atomes d'azote, chaque atome d'azote étant lié à un atome d'hydrogène, la réaction est sélective. Cela signifie que le produit subit une seule méthylation et donc que seul le produit mono-méthylé est synthétisé. Pour cela, le produit mono-méthylé est soutiré en continu, c'est-à-dire enlevé du milieu réactionnel au fur et à mesure de sa formation. Lorsque l'hétérocycle azoté contient au moins deux atomes d'azote, chacun étant lié à un atome d'hydrogène, le procédé peut être effectué en présence d'un solvant. Le solvant est choisi dans le groupe constitué par le méthoxynaphtalène, l'anisole et le trichlorobenzène.

On donne maintenant un mode de réalisation préféré de l'invention. Pour ce faire, on utilise un réacteur équipé d'un système d'agitation, d'un thermomètre et surmonté par une colonne de distillation et d'un réfrigérant.

On introduit d'abord l'hétérocycle azoté, soit seul, soit avec une partie de la quantité de carbonate de diméthyle qui sera utilisée au cours de la réaction.

Le milieu réactionnel est ensuite chauffé à une température comprise entre 100°C et 200°C, de préférence entre 120°C et 180°C.

Le procédé étant continu ou semi-continu, le carbonate de diméthyle est ensuite introduit dans le milieu réactionnel avec un débit compris entre 0,001 et 1 mol/mol de substrat.h, le substrat étant l'hétérocycle azoté.

L'hétérocycle azoté peut aussi être introduit en continu, en mélange avec du carbonate de diméthyle, avec un rapport molaire carbonate de diméthyle : hétérocycle azoté compris entre 1 et 10, de préférence entre 1 et 3.

Le méthanol produit au cours de la réaction est distillé au fur et à mesure de sa formation.

En fin de réaction, on laisse refroidir le milieu réactionnel à température ambiante et on récupère le produit méthylé.

Lorsque l'hétérocycle azoté contient au moins deux atomes d'azote, liés chacun à un atome d'hydrogène, le produit mono-méthylé formé est également soutiré au fur et à mesure de sa formation.

Les exemples qui suivent illustrent, à titre non limitatif, des variantes de mise en oeuvre de l'invention.

### Exemple 1 : synthèse du 1-méthylimidazole

La réaction est effectuée dans un réacteur de 250 ml. Le réacteur est équipé d'un agitateur, d'un thermomètre et d'un système d'alimentation tel que l'on puisse l'alimenter en continu tout au long de la réaction. Le réacteur est surmonté d'une colonne de distillation, d'une tête de reflux et d'un réfrigérant.

On introduit dans le réacteur 34,04 g d'imidazole, soit 0,5 mole. On chauffe ensuite le milieu à 170°C et on maintient cette température tout au long de la réaction.

Le carbonate de diméthyle est introduit dans le réacteur avec un débit de 145 mmoles/h, pendant 7 heures.

Le méthanol produit est distillé au fur et à mesure de sa formation. Après avoir introduit la totalité du carbonate de diméthyle, on poursuit la réaction pendant 2 heures, à 170°C. On laisse ensuite refroidir le milieu réactionnel, jusqu'à température ambiante. On récupère 33,36g de 1-méthylimidazole, soit 0,49 mole, ce qui correspond à un rendement de 98 %.

### Exemple 2 : synthèse de N-méthylmorpholine

Le montage utilisé est le même que celui décrit à l'exemple 1. On introduit 100g de 2-méthoxynaphtalène dans le réacteur puis on chauffe le milieu à 170°C et on maintient cette température tout au long de la réaction. On introduit ensuite un mélange morpholine/carbonate de diméthyle dans un rapport molaire de ½ et avec un débit en morpholine de 72 mmoles/h. La durée d'introduction du mélange morpholine/carbonate de diméthyle est de 7 heures. Ensuite, seul le carbonate de diméthyle est introduit dans le réacteur avec un débit de 100 mmoles/h pendant 2 heures. -

Le méthanol et la N-méthylmorpholine sont soutirés en continu. On obtient 22,25g de N-méthylmorpholine, soit 0,22 mol, ce qui correspond à un rendement de 43 %.

### Exemples 3 : synthèse de la N-méthylpipérazine

Le montage utilisé est le même que celui décrit à l'exemple 1. On introduit dans le réacteur 43,07g de pipérazine, soit 0,5 mole. On chauffe ensuite le milieu à 110°C et on maintient cette température tout au long de la réaction.

Le carbonate de diméthyle est introduit dans le réacteur avec un débit de 100 mmoles/h, pendant 10 heures.

Le méthanol produit est distillé au fur et à mesure de sa formation. On distille également le carbonate de diméthyle en excès, afin de stabiliser la température du milieu réactionnel à 110°C.

On laisse ensuite refroidir le milieu réactionnel, jusqu'à température ambiante. On récupère 14,52g de 1-méthylpipérazine, soit 0,145 mole, ce qui correspond à un rendement de 29 %.

### Exemple 4 : synthèse du N-méthylpyrazole

Le montage utilisé est le même que celui décrit à l'exemple 1.

On introduit dans le réacteur 20,64 g de pyrazole, soit 0,3 mole et 4,5 g de carbonate de diméthyle, soit 0,05 mole.

On chauffe ensuite le milieu à 140°C et on maintient cette température tout au long de la réaction.

Le carbonate de diméthyle est introduit dans le réacteur avec un débit de 60 mmoles/h, pendant 8 heures.

Le méthanol produit est distillé au fur et à mesure de sa formation.

On laisse ensuite refroidir le milieu réactionnel, jusqu'à température ambiante. On récupère 17,24g de N-méthylpyrazole, soit 0,21 mole, ce qui correspond à un rendement de 70 %.

### Exemple 5 : synthèse du 1,3,5-triméthylpyrazole à partir du 3,5-diméthylpyrazole

Le montage utilisé est le même que celui décrit à l'exemple 1. On introduit 24,03g de 3,5-diméthylpyrazole, soit 0,25 mole et 4,5g de carbonate de diméthyle, soit 0,05 mol.

Le milieu réactionnel est chauffé à 140°C et on maintient cette température tout au long de la réaction.

Le carbonate de diméthyle est ensuite introduit avec un débit de 50 mmol/h pendant 8 heures.

Le méthanol produit est distillé au fur et à mesure de sa formation. On distille également le carbonate de diméthyle en excès, afin de stabiliser la température du milieu réactionnel à 140°C.

Après avoir introduit la totalité du carbonate de diméthyle, on laisse refroidir le milieu réactionnel jusqu'à température ambiante. On récupère 15,41g de 1,3,5-triméthylpyrazole, soit 0,14 mol, ce qui correspond à un rendement de 57 %.

L'exemple qui suit ne fait pas partie de l'invention. Il a été réalisé dans le but de montrer que le soutirage en continu du méthanol produit au cours de la réaction est nécessaire afin de pouvoir généraliser ce procédé à plusieurs familles d'hétérocycles azotés et notamment aux hétérocycles azotés ayant un point d'ébullition inférieur à 190°C.

### Exemple 6 : Synthèse du N-méthylpyrazole sans soutirage du méthanol

Le montage utilisé est le même que celui décrit à l'exemple 1.

On introduit dans le réacteur 20,64g de pyrazole soit 0,3 mole et 4,5g de carbonate de diméthyle, soit 0,05 mole.

On chauffe ensuite le milieu à 140°C puis on introduit le carbonate de diméthyle dans le réacteur avec un débit de 60 mmoles/h (soit 5,4 g/h), pendant 8 heures. Le soutirage du méthanol formé n'a pas été effectué.

On constate que la température du milieu réactionnel est restée stable à 140°C pendant 1 heure puis a diminué progressivement jusqu'à 115°C en fin de réaction. On laisse ensuite refroidir le milieu réactionnel, jusqu'à température ambiante. On récupère seulement 3,53g de N-méthylpyrazole soit 0,042 mole, ce qui correspond à un rendement de 14%.

Le N-méthylpyrazole est donc obtenu avec un rendement nettement inférieur (14 %) à celui obtenu avec le procédé objet de l'invention, avec soutirage du méthanol (rendement de 70 %, exemple 4).

## Revendications

1. Procédé de mono-méthylation d'hétérocycles azotés contenant au moins un atome d'azote lié à un atome d'hydrogène, par réaction dudit hétérocycle avec du carbonate de diméthyle, **caractérisé en ce que** la réaction est effectuée à une température comprise entre 100°C et 200°C, à une pression comprise entre 0,93.10⁵Pa et 1,07.10⁵Pa et **en ce que** le méthanol, produit au cours de la réaction, est soutiré en continu.

2. Procédé selon la revendication 1, **caractérisé en ce que** les hétérocycles azotés ont une température d'ébullition supérieure ou égale à 120°C.

3. Procédé selon la revendication 2, **caractérisé en ce que** les hétérocycles azotés sont choisis parmi les azoles, les dérivés benzéniques des azoles, l'indoline, la pyrazolidine, la morpholine, la pipérazine et l'azépine.

4. Procédé selon la revendication 1, **caractérisé en ce que** la réaction est effectuée à une température comprise entre 120°C et 180°C.

5. Procédé selon la revendication 1, **caractérisé en ce que** la quantité de carbonate de diméthyle est comprise entre 1 et 5 moles par mole d'hétérocycle azoté.

6. Procédé selon la revendication 1, **caractérisé en ce que** le carbonate de diméthyle est ajouté progressivement dans le milieu réactionnel.

7. Procédé selon la revendication 6, **caractérisé en ce que** le carbonate de diméthyle est introduit dans le milieu réactionnel avec un débit compris entre 0,001 mol/mol d'hétérocycle azoté.h et 1 mol/mol d'hétérocycle azoté.h.

8. Procédé selon la revendication 1, **caractérisé en ce que** l'hétérocycle azoté contient au moins deux atomes d'azote liés chacun à un atome d'hydrogène.

9. Procédé selon la revendication 8, **caractérisé en ce que** l'hétérocycle azoté mono-méthylé est soutiré en continu.

## Patentansprüche

1. Verfahren zur Monomethylierung von stickstoffhaltigen Heterocyclen, enthaltend mindestens ein Stickstoffatom, das an ein Wasserstoffatom gebunden ist, durch Umsetzung des Heterocyclus mit Dimethylcarbonat, **dadurch gekennzeichnet, dass** die Umsetzung bei einer Temperatur von zwischen 100°C und 200°C und bei einem Druck von zwischen 0,93 . 10⁵ Pa und 1,07 ·10⁵ Pa durchgeführt wird, und dass das Methanol, das während der Umsetzung gebildet wird, kontinuierlich abgezogen wird.

2. Verfahren gemäß Anspruch 1, **dadurch gekennzeichnet, dass** die stickstoffhaltigen Heterocyclen einen Siedepunkt von 120°C oder höher aufweisen.

3. Verfahren gemäß Anspruch 2, **dadurch gekennzeichnet**, das die stickstoffhaltigen Heterocyclen ausgewählt sind aus Azolen, Benzolderivaten von Azolen, Indolin, Pyrazolidin, Morpholin, Piperazin und Azepin.

4. Verfahren gemäß Anspruch 1, **dadurch gekennzeichnet, dass** die Umsetzung bei einer Temperatur von zwischen 120°C und 180°C durchgeführt wird.

5. Verfahren gemäß Anspruch 1, **dadurch gekennzeichnet, dass** die Menge an Dimethylcarbonat zwischen 1 und 5 mol pro mol stickstoffhaltigem Heterocyclus liegt.

6. Verfahren gemäß Anspruch 1, **dadurch gekennzeichnet, dass** das Dimethylcarbonat fortschreitend in das Reaktionsmedium gegeben wird.

7. Verfahren gemäß Anspruch 6, **dadurch gekennzeichnet, dass** das Dimethylcarbonat mit einem Durchsatz von zwischen 0,001 mol/mol stickstoffhaltigem Heterocyclus · h und 1 mol/mol stickstoffhaltigem Heterocyclus · h in das Reaktionsmedium gegeben wird.

8. Verfahren gemäß Anspruch 1, **dadurch gekennzeichnet, dass** der stickstoffhaltige Heterocyclus mindestens zwei Atome Stickstoff enthält, die jeweils an ein Wasserstoffatom gebunden sind.

9. Verfahren gemäß Anspruch 8, **dadurch gekennzeichnet, dass** der monomethylierte stickstoffhaltige Heterocyclus kontinuierlich abgezogen wird.

## Claims

1. Process for the monomethylation of nitrogenous heterocycles comprising at least one nitrogen atom bonded to a hydrogen atom by reaction of said heterocycle with dimethyl carbonate, **characterized in that** the reaction is carried out at a temperature of between 100°C and 200°C, at a pressure of between 0.93 x 10⁵ Pa and 1.07 × 10⁵ Pa, and the methanol produced during the reaction is continuously withdrawn.

2. Process according to Claim 1, **characterized in that** the nitrogenous heterocycles have a boiling point of greater than or equal to 120°C.

3. Process according to Claim 2, **characterized in that** the nitrogenous heterocycles are chosen from azoles, benzene derivatives of azoles, indoline, pyrazolidine, morpholine, piperazine and azepine.

4. Process according to Claim 1, **characterized in that** the reaction is carried out at a temperature of between 120°C and 180°C.

5. Process according to Claim 1, **characterized in that** the amount of dimethyl carbonate is between 1 and 5 mol per mole of nitrogenous heterocycle.

6. Process according to Claim 1, **characterized in that** the dimethyl carbonate is added gradually to the reaction medium.

7. Process according to Claim 6, **characterized in that** the dimethyl carbonate is introduced into the reaction medium with a flow rate of between 0.001 mol/mol of nitrogenous heterocycle.h and 1 mol/mol of nitrogenous heterocycle.h.

8. Process according to Claim 1, **characterized in that** the nitrogenous heterocycle comprises at least two nitrogen atoms each bonded to a hydrogen atom.

9. Process according to Claim 8, **characterized in that** the monomethylated nitrogenous heterocycle is continuously withdrawn.
